# EUROPEAN PATENT APPLICATION

(11) **EP 4 768 024 A1**
(43) Date of publication of application: **01.07.2026**
(21) Application number: 25210101.9
(22) Date of filing: 21.10.2025
(51) Int. Cl.: A61K 9/48, A61K 31/165

(54) **MINI-TABLETS IN CAPSULE FORMULATIONS OF LISDEXAMFETAMINE DIMESYLATE**

(30) Priority: 20.03.2025 TR 2025033410; 24.12.2024 TR 2024202870
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); BENGUL, Furkan, Istanbul (TR); SARP, Onder, Istanbul (TR); AKYEL, Fehimenur, Istanbul (TR)
(74) Representative: Genç Ilhan, Oznur

(57) **Abstract**

The present invention relates to mini-tablets in capsule formulation comprising lisdexamfetamine dimesylate and at least one stabilizer. Furthermore, the invention discloses a process which is simple, rapid, cost effective, time-saving and industrially convenient process and is created to eliminate the disadvantages of active agent.

## Description

### Field of the Invention

The present invention relates to mini-tablets in capsule formulation comprising lisdexamfetamine dimesylate and at least one stabilizer. Furthermore, the invention discloses a process which is simple, rapid, cost effective, time-saving and industrially convenient process and is created to eliminate the disadvantages of active agent.

### Background of the Invention

Lisdexamfetamine dimesylate is used to treat attention deficit hyperactivity disorder (ADHD) in adults and children 6 years of age and older. This medicine is also used to treat moderate to severe binge eating disorder (BED). It belongs to the group of medicines called central nervous system (CNS) stimulants.

Lisdexamfetamine dimesylate increases attention and decreases restlessness in children and adults who are overactive, cannot concentrate for very long, or are easily distracted and impulsive. This medicine is used as part of a total treatment program that also includes social, educational, and psychological treatment.

Lisdexamfetamine dimesylate is also referred to as I-lysine-d-amphetamine dimesylate of Formula I is chemically named as (2S)-2,6-Diamino-N-[(1S)-1-methyl-2-phenylethyl]-hexanamide dimesylate.

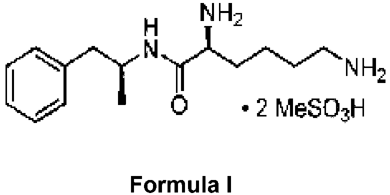

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is currently commercially available only as 10, 20, 30, 40, 50, 60 and 70 mg hard gelatine capsules. Each capsules contains 10, 20, 30, 40, 50, 60 and 70 mg lisdexamfetamine dimesylate, equivalent to 5.8 mg, 11.6 mg, 17.3 mg, 23.1 mg, 28.9 mg, 34.7 mg, 40.5 mg lisdexamfetamine, respectively.

The patent WO2006121552A2 discloses a capsule formulation containing lisdexamfetamine dimesylate, microcrystalline cellulose, croscarmellose sodium, magnesium stearate.

The formulation comprising the capsule form of lisdexamfetamine dimesylate is available in the prior art. However, there is still a need for a high stability formulation to be developed taking into account the disadvantages of lisdexamfetamine dimesylate. Lisdexamfetamine dimesylate is an active substance that is deliquescent and needs to be formulated accordingly. By using lisdexamfetamine dimesylate with a stabilizer in the form of mini-tablets in capsule, we have obtained a formulation that reduces moisture absorption and thus has high stability.

### Detailed Description of the Invention

The main object of the present invention is to provide a capsule formulation comprising lisdexamfetamine dimesylate with high stability.

Another object of the present invention is to provide mini-tablets in capsule formulation comprising lisdexamfetamine dimesylate having the desired dissolution profile and content uniformity.

Another object of the present invention is to provide a process for mini-tablets in capsule formulation comprising lisdexamfetamine dimesylate prepared by simple, easy, time-saving and fast manufacturing process.

The term "mini tablet", as used herein, refers to small tablets with a diameter equal to or less than 4 mm that are typically filled into a capsule. Thickness of this mini tablets equal to or less than 3 mm. The mini tablets have round shape and smooth surface to ease coating process.

Mini tablets have many advantages. Some benefits of mini-tablets include excellent size uniformity, regular shape and a smooth surface. Mini tablets also help with content uniformity.

High humidity in the environment may affect the uniformity of dosage form and stability of the product. Therefore, it is important that lisdexamfetamine dimesylate is protected from moisture to maintain its stability. We aimed to reduce the moisture absorption capacity of the dosage form and increase the stability of lisdexamfetamine dimesylate by using stabilizers in the formulation. Surprisingly, we found that moisture absorption was reduced when stabilizer was used, thus obtaining a formulation with high stability and the desired dissolution profile.

According to one embodiment of the present invention, mini-tablets in capsule formulation comprising lisdexamfetamine dimesylate wherein comprising at least one stabilizer.

According to one embodiment of the present invention, mini-tablets are filled into the capsule wherein mini-tablets have at least one coating. In this invention, a smooth and strong form which provides high stability mechanically and chemically has been achieved for lisdexamfetamine dimesylate.

According to one embodiment of the present invention, the amount of the lisdexamfetamine dimesylate is between 30.0% and 45.0% by weight of the total formulation. The amount of the lisdexamfetamine dimesylate is between 30.0% and 40.0% by weight of the total formulation.

Suitable stabilizers are selected from the group comprising magnesium aluminometasilicate, glycerol dibehenate, isomalt, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

According to one embodiment of the present invention, the stabilizer is magnesium aluminometasilicate or glycerol dibehenate or mixture thereof.

According to one embodiment of the present invention, the stabilizer is magnesium aluminometasilicate.

According to one embodiment of the present invention, the stabilizer is glycerol dibehenate. It is also known that glycerol dibehenate is used as a moisture protective agent.

According to one embodiment of the present invention, the amount of the stabilizer is between 0.1% and 10.0% by weight of the total formulation. Preferably, it is between 2.5% and 9.0% by weight in the total formulation.

According to one embodiment of the present invention, the capsule formulation further comprises at least one pharmaceutically acceptable excipient selected from the group comprising fillers, disintegrants, binders, glidants/lubricants or mixtures thereof.

Suitable fillers are selected from the group comprising microcrystalline cellulose, lactose, starch, sucrose, ammonium alginate, calcium phosphate, dibasic calcium phosphate, neutral pellets, calcium sulfate, cellulose acetate, erythritol, ethyl cellulose, fructose, glyceryl palmitostearate, lactose, mannitol, magnesium carbonate, magnesium oxide, maltodextrin, maltose, medium chain triglycerides, polydextrose, polymethacrylates, sodium chloride, sorbitol, sugar spheres, tragacanth, trehalose, polysorbate 80, xylitol or mixtures thereof.

According to this embodiment of the present invention, the filler is microcrystalline cellulose.

According to this embodiment of the present invention, the amount of filler is between 35.0% and 60.0% by weight of the total formulation, preferably it is between 38.0% and 57.0% by weight of the total formulation.

Suitable disintegrants are selected from the group comprising croscarmellose sodium, sodium starch glycolate, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

According to one embodiment of the present invention, the disintegrant is croscarmellose sodium or sodium starch glycolate or low-substituted hydroxypropyl cellulose or crospovidone or mixtures thereof.

According to one embodiment of the present invention, the amount of disintegrant is between 0.1% and 10.0% by weight of the total formulation, preferably it is between 2.0% and 8.0% by weight of the total formulation.

Suitable binders in the mixture are selected from the group comprising sodium carboxymethyl cellulose, polyvinylpyrrolidone (K30, K90), sodium carboxymethyl cellulose, polyethylene glycol, starch, pregelatinized starch, sodium alginate, hydroxypropyl methyl cellulose, hydroxypropyl cellulose, carboxy methyl cellulose, methyl cellulose, gelatin, carrageenan, guar gum, polymethacrylates, methacrylate polymers, hyaluronic acid, pectin, polysaccharides, carbomer, poloxamer, polyacrylamide, aluminium hydroxide, polyoxyethylene-alkyl ether, polydextrose, polyethylene oxide or mixtures thereof.

According to one embodiment of the present invention, the binder is polyvinylpyrrolidone or hydroxypropyl cellulose or mixtures thereof.

According to one embodiment of the present invention, the amount of binder is between 0.5% and 8.0% by weight in the total formulation.

Suitable glidants/lubricants are selected from the group comprising sodium stearyl fumarate, magnesium stearate, talc, colloidal silicon dioxide, corn, calcium stearate, zinc stearate, magnesium lauryl sulfate, sodium oleate, sodium acetate, stearic acid, fumaric acid, or mixtures thereof.

According to one embodiment of the present invention, the glidant/lubricant is magnesium stearate or colloidal silicon dioxide or mixtures thereof.

According to one embodiment of the present invention, the amount of glidant/lubricant is between 0.1% and 3.0% by weight in the total formulation.

According to one embodiment of the present invention, mini-tablets in capsule formulation comprises;
- Lisdexamfetamine dimesylate
- Microcrystalline cellulose
- Colloidal silicon dioxide

According to one embodiment of the present invention, mini-tablets in capsule formulation comprises;
- Lisdexamfetamine dimesylate
- Microcrystalline cellulose
- Colloidal silicon dioxide
- Glycerol dibehenate

According to one embodiment of the present invention, mini-tablets are obtained with dry granulation. Used granules provide higher flowability and higher flowability for tablet compression and dose-proportionality between doses therefore a simple and low-cost production method is employed.

According to this embodiment of the invention, a process for mini-tablets in capsule formulations comprises the following steps:
a. Sieving and blending lisdexamfetamine dimesylate and at least one stabilizer,
b. Sieving at least one filler and at least one disintegrant and at least one binder and at least one glidants/lubricants and blending them with the powder mixture obtained in (a),
c. Compressing the mixture into mini-tablet form and then coating,
d. Filling the coated mini-tablets into capsules.

### Example 1;

| **Ingredient** | **%by weight** | **%by weight** |
|---|---|---|
| Lisdexamfetamine Dimesylate | 33.33 | 30-50 |
| Microcrystalline Cellulose | 56.00 | 35-60 |
| Povidone | 1.67 | 1-20 |
| Crospovidone | 4.00 | 0.1-10 |
| Glycerol Dibehenate | 4.33 | 0.1-5 |
| Colloidal Silicon Dioxide | 0.67 | 0.1-5 |
| **Core Tablet Weight** | **100** | **100** |
| **Opadry AMB II** | 3.00 | 1-5 |
| *Polyvinyl Alcohol - Part. Hydrolyzed | | |
| *Talc | | |
| *Titanium Dioxide | | |
| *GMDCC, GMCC Type 1, Glycerol of Fatty Acids | | |
| *Sodium Lauryl Sulfate | | |
| *Colorant | | |
| **Coated Tablet Weight** | | |
| **Gelatin Capsule** | | |

a. Sieving and blending lisdexamfetamine dimesylate and 80% of glycerol dibehenate,
b. Sieving microcrystalline cellulose, povidone, half of crospovidone and half of colloidal silicon dioxide and blending them with the powder mixture obtained in step(a),
c. Compacting the mixture and then sieving,
d. Sieving the remaining amount of glycerol dibehenate, the remaining amount of crospovidone CL and the remaining amount of colloidal silicon dioxide and then adding into the mixture at step(c),
e. Compressing the mixture into mini-tablet form and then coating,
f. Filling the coated mini-tablets into capsules.

### Example 2;

| **Ingredient** | **%by weight** | **%by weight** |
|---|---|---|
| Lisdexamfetamine Dimesylate | 33.33 | 30-50 |
| Microcrystalline Cellulose | 53.67 | 40-60 |
| Hydroxypropyl Cellulose | 1.67 | 1-20 |
| Low Substituted Hydroxypropyl Cellulose | 7.07 | 0.1-10 |
| Glycerol Dibehenate | 3.57 | 0.1-5 |
| Colloidal Silicon Dioxide | 0.70 | 0.1-5 |
| **Core Tablet Weight** | **100** | **100** |
| **Opadry AMB II** | 3.00 | 1-5 |
| *Polyvinyl Alcohol - Part. Hydrolyzed | | |
| *Talc | | |
| *Titanium Dioxide | | |
| *GMDCC, GMCC Type 1, Glycerol of Fatty Acids | | |
| *Sodium Lauryl Sulfate | | |
| *Colorant | | |
| **Coated Tablet Weight** | | |
| **Gelatin Capsule** | | |

a. Sieving and blending lisdexamfetamine dimesylate and 80% of glycerol dibehenate and 20% of microcrystalline cellulose,
b. Sieving 80% of microcrystalline cellulose, hydroxypropyl cellulose, 50% of low substituted hydroxypropyl cellulose, 50% of colloidal silicon dioxide and blending them with the powder mixture obtained in step(a),
c. Compacting the mixture and then sieving,
d. Sieving the remaining amount of glycerol dibehenate, the remaining amount of low substituted hydroxypropyl cellulose and the remaining amount of colloidal silicon dioxide and then adding into the mixture at step(c),
e. Compressing the mixture into mini-tablet form and then coating,
f. Filling the coated mini-tablets into capsules.

### Example 3;

| **Ingredient** | **%by weight** | **%by weight** |
|---|---|---|
| Lisdexamfetamine Dimesylate | 33.33 | 30-50 |
| Microcrystalline Cellulose | 40.00 | 40-60 |
| Dibasic Calcium Phosphate (Anhydrous) | 16.67 | 1-20 |
| Povidone | 1.83 | 0.1-10 |
| Sodium Starch Glycolate | 4.00 | 0.1-5 |
| Glycerol Dibehenate | 4.00 | 0.1-5 |
| Colloidal Silicon Dioxide | 0.17 | 0.1-5 |
| **Core Tablet Weight** | **100** | **100** |
| **Opadry AMB II** | 3.00 | 1-5 |
| *Polyvinyl Alcohol - Part. Hydrolyzed | | |
| *Talc | | |
| *Titanium Dioxide | | |
| *GMDCC, GMCC Type 1, Glycerol of Fatty Acids | | |
| *Sodium Lauryl Sulfate | | |
| *Colorant | | |
| **Coated Tablet Weight** | | |
| **Gelatin Capsule** | | |

a. Sieving and blending lisdexamfetamine dimesylate and 80% of glycerol dibehenate and 20% of microcrystalline cellulose,
b. Sieving 80% of microcrystalline cellulose, povidone, sodium starch glycolate, dibasic calcium phosphate and blending them with the powder mixture obtained in step(a),
c. Adding colloidal silicon dioxide and mixing,
d. Sieving the remaining amount of glycerol dibehenate and then adding into the mixture at step(c),
e. Compressing the mixture into mini-tablet form and then coating,
f. Filling the coated mini-tablets into capsules.

### Example 4;

| **Ingredient** | **%by weight** | **%by weight** |
|---|---|---|
| Lisdexamfetamine Dimesylate | 33.33 | 30-50 |
| Microcrystalline Cellulose | 52.33 | 40-60 |
| Povidone | 1.67 | 1-20 |
| Croscarmellose Sodium | 4.00 | 0.1-10 |
| Magnesium Aluminometasilicate | 8.17 | 0.1-10.0 |
| Colloidal Silicon Dioxide | 0.17 | 0.1-5 |
| Magnesium Stearate | 0.33 | 0.1-5 |
| **Core Tablet Weight** | **100** | **100** |
| **Opadry AMB II** | 3.00 | 1-5 |
| *Polyvinyl Alcohol - Part. Hydrolyzed | | |
| *Talc | | |
| *Titanium Dioxide | | |
| *GMDCC, GMCC Type 1, Glycerol of Fatty | | |
| Acids | | |
| *Sodium Lauryl Sulfate | | |
| *Colorant | | |
| **Coated Tablet Weight** | | |
| **Gelatin Capsule** | | |

a. Sieving and blending lisdexamfetamine dimesylate and magnesium aluminometasilicate,
b. Sieving microcrystalline cellulose, croscarmellose sodium, povidone and blending them with the powder mixture obtained in step(a),
c. Adding colloidal silicon dioxide and mixing,
d. Sieving the magnesium stearate and then adding into the mixture at step(c),
e. Compressing the mixture into mini-tablet form and then coating,
f. Filling the coated mini-tablets into capsules.

## Claims

1. Mini-tablets in capsule formulation comprising lisdexamfetamine dimesylate wherein comprising at least one stabilizer.

2. The mini-tablets in capsule formulation according to claim 1, wherein the amount of the lisdexamfetamine dimesylate is between 30.0% and 45.0% by weight of the total formulation.

3. The mini-tablets in capsule formulation according to claim 1, wherein stabilizers are selected from the group comprising magnesium aluminometasilicate, glycerol dibehenate, isomalt, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

4. The mini-tablets in capsule formulation according to claim 3, wherein the stabilizer is magnesium aluminometasilicate or glycerol dibehenate or mixture thereof.

5. The mini-tablets in capsule formulation according to claim 3 or 4, wherein the amount of the stabilizer is between 0.1% and 10.0% by weight of the total formulation.

6. The mini-tablets in capsule formulation according to any preceding claim, wherein further comprises at least one pharmaceutically acceptable excipient selected from the group comprising fillers, disintegrants, binders, glidants/lubricants or mixtures thereof.

7. The mini-tablets in capsule formulation according to claim 6, wherein fillers are selected from the group comprising microcrystalline cellulose, lactose, starch, sucrose, ammonium alginate, calcium phosphate, dibasic calcium phosphate, neutral pellets, calcium sulfate, cellulose acetate, erythritol, ethyl cellulose, fructose, glyceryl palmitostearate, lactose, mannitol, magnesium carbonate, magnesium oxide, maltodextrin, maltose, medium chain triglycerides, polydextrose, polymethacrylates, sodium chloride, sorbitol, sugar spheres, tragacanth, trehalose, polysorbate 80, xylitol or mixtures thereof.

8. The mini-tablets in capsule formulation according to claim 6, wherein disintegrants are selected from the group comprising croscarmellose sodium, sodium starch glycolate, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

9. The mini-tablets in capsule formulation according to claim 6, wherein binders in the mixture are selected from the group comprising sodium carboxymethyl cellulose, polyvinylpyrrolidone (K30, K90), sodium carboxymethyl cellulose, polyethylene glycol, starch, pregelatinized starch, sodium alginate, hydroxypropyl methyl cellulose, hydroxypropyl cellulose, carboxy methyl cellulose, methyl cellulose, gelatin, carrageenan, guar gum, polymethacrylates, methacrylate polymers, hyaluronic acid, pectin, polysaccharides, carbomer, poloxamer, polyacrylamide, aluminium hydroxide, polyoxyethylene-alkyl ether, polydextrose, polyethylene oxide or mixtures thereof.

10. The mini-tablets in capsule formulation according to claim 6, wherein glidants/lubricants are selected from the group comprising sodium stearyl fumarate, magnesium stearate, talc, colloidal silicon dioxide, corn, calcium stearate, zinc stearate, magnesium lauryl sulfate, sodium oleate, sodium acetate, stearic acid, fumaric acid, or mixtures thereof.

11. A process for mini-tablets in capsule formulations according to any of the preceding claims comprises the following steps:
a) Sieving and blending lisdexamfetamine dimesylate and at least one stabilizer,
b) Sieving at least one filler and at least one disintegrant and at least one binder and at least one glidants/lubricants and blending them with the powder mixture obtained in (a),
c) Compressing the mixture into mini-tablet form and then coating,
d) Filling the coated mini-tablets into capsules.
